(19) Europäisches Patentamt
European Patent Office
Office européen des brevets

(11) Numéro de publication: **0 460 996 B1**

(12) **FASCICULE DE BREVET EUROPEEN**

(45) Date de publication de fascicule du brevet: **14.09.94**

(51) Int. Cl.5: **A61K 7/13**

(21) Numéro de dépôt: **91401371.9**

(22) Date de dépôt: **29.05.91**

# (54) Procédé de teinture de fibres kératiniques avec un aminoindole associé à un dérivé quinonique.

(30) Priorité: **29.05.90 FR 9006660**

(43) Date de publication de la demande:
**11.12.91 Bulletin 91/50**

(45) Mention de la délivrance du brevet:
**14.09.94 Bulletin 94/37**

(84) Etats contractants désignés:
**AT BE CH DE DK ES FR GB GR IT LI NL SE**

(56) Documents cités:
**EP-A- 0 376 776**
**FR-A- 2 626 173**

(73) Titulaire: **L'OREAL**
**14, Rue Royale**
**F-75008 Paris (FR)**

(72) Inventeur: **Baudry, Alain**
**19 Villa du Buisson Ardent**
**F-95500 Gonesse (FR)**
Inventeur: **Junino, Alex**
**16, rue Docteur Bergonié**
**F-93180 Livry-Gargan (FR)**
Inventeur: **Richard, Hervé**
**48, rue de l'Ermitage**
**F-75020 Paris (FR)**

(74) Mandataire: **Casalonga, Axel et al**
**BUREAU D.A. CASALONGA - JOSSE**
**Morassistrasse 8**
**D-80469 München (DE)**

EP 0 460 996 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention est relative à un nouveau procédé de coloration des fibres kératiniques et plus particulièrement des fibres kératiniques humaines, telles que les cheveux, mettant en oeuvre au moins un aminoindole, associé à au moins un dérivé quinonique.

On a déjà décrit, notamment dans les brevets français FR-A-1 133 594, 1 166 172 et 2 390 158 de teindre les fibres kératiniques et en particulier les cheveux, à l'aide du 5,6-dihydroxyindole ou de ses dérivés

Les demandes de brevets 2 536 993 et 2 594 331 proposent des procédés de teinture avec le 5,6-dihydroxyindole eu utilisant soit des cations métalliques jouant le rôle de promoteur de mélanogénèse, soit du manganèse sous forme de permanganate ou encore du bichromate. La demanderesse a, par ailleurs, décrit dans ses brevets FR-A 2 593 061, 2 593 062 un procédé de teinture mettant en oeuvre le 5,6-dihydroxyindole en association avec des ions iodure, ces procédés nécessitant l'utilisation de peroxyde d'hydrogène.

La demande EP-A 271 186 est relative à des procédés de teinture des cheveux utilisant des dérivés d'hydroxy indoles en association avec des systèmes oxydants tels que l'acide périodique et ses sels hydrosolubles, l'oxyde d'argent ou de plomb, le réactif de Fenton, le sulfate de césium, le persulfate d'ammonium, l'hypochlorite de sodium, le chlorure ferrique ou le ferricyanure de potassium.

La demande EP-A-376 776 est relative à un procédé de teinture des fibres kératiniques qui consiste à imprégner lesdites fibres à l'aide d'un mono ou dihydroxyindole ; cette imprégnation étant précédée ou suivie par l'application d'une composition contenant un dérivé quinonique à titre d'agent oxydant.

La demanderesse a découvert, ce qui fait l'objet de l'invention, qu'il était possible, de façon surprenante, d'effectuer une teinture à l'aide d'un amino indole, en imprégnant les fibres kératiniques et en particulier les cheveux par l'aminoindole, cette imprégnation étant précédée ou suivie par l'application d'une composition contenant un dérivé quinonique à titre d'agent oxydant.

La demanderesse a découvert, notamment qu'il était possible de teindre les cheveux naturels dans des tons allant du châtain clair au noir avec des reflets variés allant du rouge au bleu.

L'invention a donc pour objet un nouveau procédé de teinture des fibres kératiniques et en particulier des fibres kératiniques humaines, mettant en oeuvre un aminoindole et à titre d'agent oxydant un dérivé quinonique.

D'autres objets de l'invention sont constitués par des dispositifs à plusieurs compartiments utilisés dans le cadre de ce procédé.

D'autres objets de l'invention apparaîtront à la lecture de la description et des exemples qui suivent.

Le procédé de teinture des fibres kératiniques et en particulier des fibres kératiniques humaines, telles que les cheveux, conforme à l'invention, est essentiellement caractérisé par le fait que l'on applique sur ces fibres au moins une composition (A) contenant, dans un milieu approprié pour la teinture, au moins un aminoindole, l'application de la composition (A) étant précédée ou suivie par l'application d'une composition (B) contenant, dans un milieu approprié pour la teinture, au moins un dérivé quinonique choisi parmi les ortho- ou parabenzoquinones, les ortho- ou parabenzoquinones monoimines ou diimines, les ortho-ou parabenzoquinones sulfonimides, les , -alkylène bis-1,4-benzoquinones, les 1,2- ou 1,4-naphtoquinones, les 1,2- ou 1,4-naphtoquinone-monoimines ou diimines; les aminoindoles et les dérivés quinoniques étant choisis de façon à ce que la différence de potentiel d'oxydoréduction E entre le potentiel d'oxydoréduction $E_i$ des aminoindoles, déterminé à pH 7 en milieu phosphate sur électrode de carbone vitreux, par voltamétrie, et le potentiel d'oxydoréduction $E_q$ des dérivés quinoniques déterminé à pH 7 en milieu phosphate par polarographie sur électrode de mercure et par rapport à l'électrode au calomel saturé, soit tel que

$$\Delta E = E_i - E_q \leq 470 \text{ mV}.$$

Les aminoindoles utilisés conformément à l'invention, répondent à la formule (I) suivante :

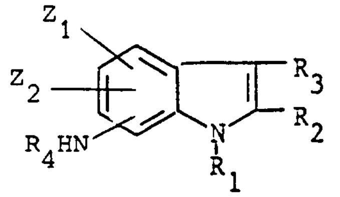

(I)

dans laquelle :

$R_1$ désigne un atome d'hydrogène ou un groupement alkyle en $C_1$-$C_4$ linéaire ou ramifié;

$R_2$ et $R_3$, indépendamment l'un de l'autre, désignent un atome d'hydrogène, un groupement alkyle en $C_1$-$C_4$, un groupement carboxyle ou un groupement alcoxy ($C_1$-$C_4$) carbonyle;

$R_4$ désigne un atome d'hydrogène ou un groupement alkyle en $C_1$-$C_4$ ou hydroxyalkyle en $C_1$-$C_4$ ou polyhydroxyalkyle en $C_2$-$C_4$, acétyle, aminoalkyle en $C_1$-$C_6$ dont l'amine peut être éventuellement mono ou disubstituée par un alkyle en $C_1$-$C_4$;

$Z_1$ et $Z_2$, identiques ou différents, représentent un atome d'hydrogène, un groupement alkyle en $C_1$-$C_4$, un hydroxy, un halogène ou alcoxy en $C_1$-$C_4$;

le groupement $R_4NH$ occupant les positions 4, 5, 6 ou 7 du noyau benzénique, ainsi que leurs sels.

Les composés de formule (I) dans laquelle $R_4$ est différent d'un atome d'hydrogène sont désignés par la formule (IA) et peuvent être préparés selon le schéma réactionnel ci-après.

Le composé (IA) est obtenu à partir du composé (IB) ($R_4$ = H) par les méthodes de substitution des amines aromatiques selon le schéma réactionnel suivant :

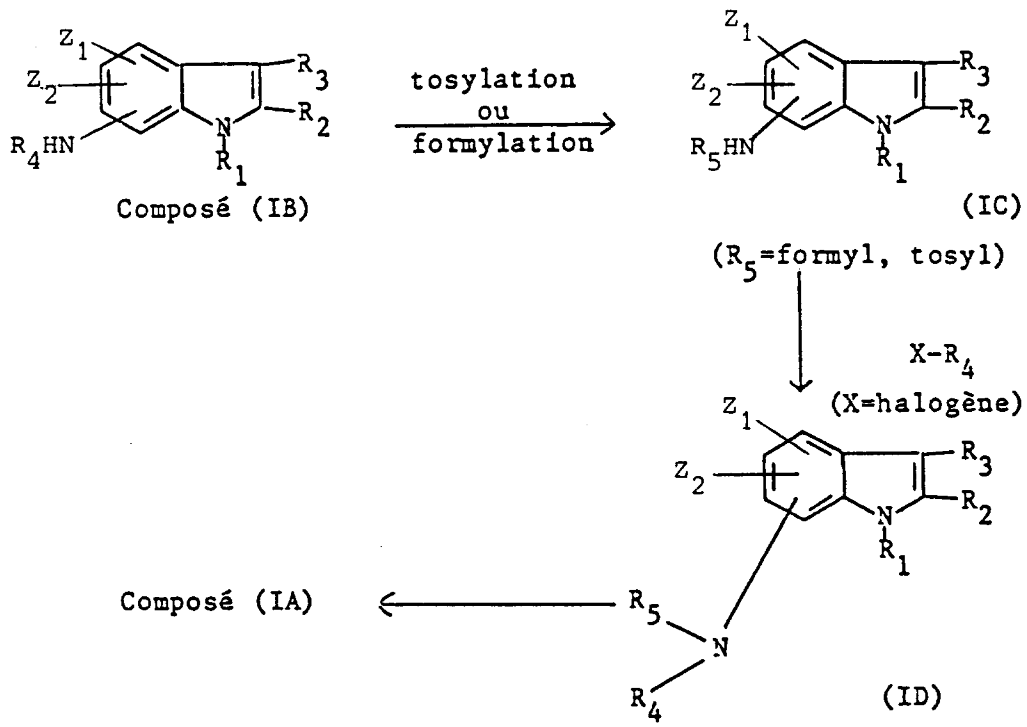

Composé (IB)
(IC)
($R_5$=formyl, tosyl)
Composé (IA)
(ID)

Par formylation ou tosylation, on obtient le composé (IC). Le composé (IC) est alkylé dans un deuxième temps par l'halogénure d'alkyle X-$R_4$. Lorsque l'halogénure d'alkyle est utilisé en excès, un second groupement $R_4$ est introduit. On obtient le produit (IA) par déformylation ou détosylation du composé (ID).

Parmi les méthodes d'hydroxylalkylation, on peut citer, par exemple, l'action du chloroformiate de $\beta$-chloréthyle sur le composé (IB) qui permet d'obtenir dans un premier temps, le carbamate de $\beta$-chloréthyle correspondant qui, soumis dans un deuxième temps à l'action d'une base minérale forte, permet d'obtenir le composé (IA) pour lequel le radical $R_4$ est un radical $\beta$-hydroxyéthyle.

Les composés particulièrement préférés, selon l'invention, sont choisis parmi :

le 5-aminoindole, le 6-aminoindole, le 7-aminoindole, le 6-N-$\beta$-hydroxyéthylaminoindole, le 6-N-$\beta$-hydroxyéthylamino 1-méthylindole, le 6-méthyl aminoindole, le 6-amino N-méthylindole, le 6-amino 2-carboxyindole, le 4-amino 2,3-diméthylindole, le 6-amino 2,3-diméthylindole, le 7-amino 2,3-diméthylindole, le 6-amino 3-éthyl 2-méthylindole, le 7-amino 3-éthyl 2-méthylindole, le 6-amino 3-méthylindole, le 6-amino 2-méthylindole, le 6-amino 2-éthoxycarbonylindole, le 4-aminoindole, le 5-amino 6-méthoxy 2,3-diméthylindole, le 6-amino 5-méthoxy 2,3-diméthylindole, le 5-amino 6-hydroxy 2,3-diméthylindole, le 6-amino 5-hydroxy 2,3-diméthylindole, le 5-amino N-méthylindole, le 6-N-($\beta$,$\gamma$-dihydroxypropyl)aminoindole, le 6-amino 2,3,4,5-tétraméthylindole, le 6-amino 5-chloro 2,3-diméthylindole, le 6-amino 5-éthyl 2,3-diméthylindole, le 6-amino 2,3,4-triméthylindole,le 6-amino 5-hydroxy 2-méthylindole, le 4-méthylaminoindole, le 4-amino N-méthylindole, le 6-amino 2,3,7-triméthylindole, le 6-amino 2,3,5-triméthylindole, le 5-acétylamino 6-méthoxy 2,3-diméthylindole, le 6-amino 4-méthylindole, le 6-amino 5-méthylindole, le 4-amino 7-méthylindole, le 6-amino

7-éthyl 3-méthylindole, le 6-amino 5,7-diméthylindole, le 6-amino 5,7-diéthylindole, le 7-amino 5-méthyl 2-éthoxycarbonylindole, le 7-amino 5-chloro 2-éthoxycarbonylindole, le 7-amino 5-éthoxy 2-éthoxycarbonylindole, le 7-amino 5-méthoxy 2-éthoxycarbonylindole, le 7-(4'-diméthylamino 1'-méthylbutyl)amino 5-méthoxyindole, le 7-(4'-diméthylaminobutyl)amino 5-méthoxyindole, le 6-amino 5-fluoroindole, le 6-amino 5-fluoro 1-sec.butylindole, le 6-amino 5-fluoro 1-n-propylindole, le 6-amino 2-méthoxy carbonyl 5-méthoxy N-méthylindole, le 6-amino 5-méthoxy 2-méthoxycarbonylindole, le 6-amino 5-méthoxy 2-éthoxycarbonylindole, le 6-amino 5-méthoxy 2-carboxyindole, le 6-amino 5-hydroxy 1,2-diméthylindole et le 6-amino 4-méthoxy 2-méthoxycarbonylindole.

Les dérivés quinoniques sont choisis plus particulièrement parmi les composés répondant aux formules (II) et (II')) :

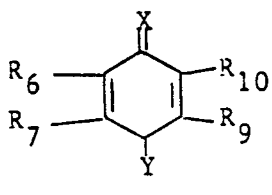

(II)

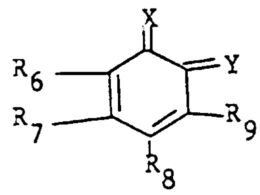

(II')

dans lesquelles :

X désigne oxygène ou un groupement $NR_{11}$;

Y désigne oxygène ou un groupement $NR_{12}$;

$R_{11}$ et $R_{12}$, identiques ou différents, désignant hydrogène, halogène, un radical alkyle en $C_1$-$C_4$, hydroxyalkyle en $C_1$-$C_4$, un radical alkyl ($C_1$-$C_4$) sulfonyle ou un radical phénylsulfonyle;

$R_6$, $R_7$, $R_8$, $R_9$ et $R_{10}$ désignent, indépendamment l'un de l'autre, hydrogène, un radical alkyle ($C_1$-$C_4$), un groupement carboxyle, alkyl ($C_1$-$C_4$) carbonyle, alcoxy ($C_1$-$C_4$) carbonyle, alcoxy ($C_1$-$C_4$) méthyle, alkyl ($C_1$-$C_4$) thiométhyle, hydroxyalkyl ($C_1$-$C_4$) thiométhyle, hydroxyalkyl ($C_1$-$C_4$) sulfinyle,

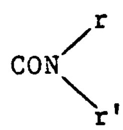

(r et r' désignant, indépendamment l'un de l'autre,
hydrogène ou alkyle en $C_1$-$C_4$), carboxyalkyle en $C_1$-$C_4$, halogène, hydroxyalkyl ($C_1$-$C_4$), amino substitué ou non par un ou deux groupes alkyle ou hydroxyalkyle en $C_1$-$C_4$, acyl ($C_2$-$C_6$) amino, $SO_3M$, où M désigne hydrogène, K ou Na, un groupement sulfoxyde, sulfone ou sulfonamide, éventuellement substitué, ou bien un radical $OZ_1$ dans lequel $Z_1$ peut être hydrogène, alkyl ($C_1$-$C_4$), hydroxyalkyl ($C_1$-$C_4$), carboxyalkyl ($C_1$-$C_4$), phényle éventuellement substitué par alcoxy en $C_1$-$C_4$, ou encore un radical $-SZ_2$ dans lequel $Z_2$ est un groupe alkyle en $C_1$-$C_4$, hydroxyalkyle en $C_1$-$C_4$, dihydroxyalkyle en $C_2$-$C_4$, carboxyalkyle en $C_1$-$C_4$;

$R_6$ et $R_7$ pouvant former avec les atomes de carbone auxquels ils sont rattachés, le groupement cyclique suivant :

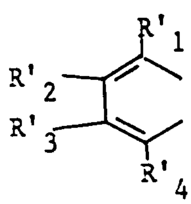

(III)

dans lequel :

$R'_1$, $R'_2$, $R'_3$ et $R'_4$ ont les significations indiquées ci-dessus pour $R_6$, $R_7$, $R_8$, $R_9$, $R_{10}$, lorsqu'ils ne forment pas de cycles.

Conformément à l'invention, halogène est choisi de préférence parmi le fluor, le chlore ou le brome.

Les composés préférés sont choisis parmi les dérivés quinoniques dont le potentiel $E_q$ est supérieur ou égal à -220 mV et notamment parmi les benzoquinones de formules (II) et (II'), dans lesquelles :

$R_6$, $R_7$, $R_8$, $R_9$, $R_{10}$ désignent, indépendamment l'un de l'autre, hydrogène, alkyle inférieur en $C_1$-$C_4$, alcoxy inférieur en $C_1$-$C_4$, halogène, acylamino en $C_2$-$C_6$, $SO_3M$, alcoxy ($C_1$-$C_4$) méthyle, carboxy alkyle ($C_1$-$C_4$), alcoxy ($C_1$-$C_4$) carbonyle, dialkyl ($C_1$-$C_4$) amino, $OZ_1$ dans lequel $Z_1$ représente carboxylalkyl ($C_1$-$C_4$), hydroxyalkyl ($C_1$-$C_4$), $SZ_2$ dans lequel $Z_2$ représente alkyl ($C_1$-$C_4$), hydroxyalkyl ($C_1$-$C_4$), dihydroxyalkyl ($C_2$-$C_4$), carboxyalkyl ($C_1$-$C_4$).

X désigne oxygène ou le groupe $NR_{11}$;

Y désigne oxygène ou le groupement $NR_{12}$,

$R_{11}$ et $R_{12}$, indépendamment l'un de l'autre, désignent hydrogène, halogène ou alkyle inférieur en $C_1$-$C_4$; méthylsulfonyle ou phénylsulfonyle.

D'autres composés préférés utilisés conformément à l'invention, répondent aux formules (V) et (VI) ci-après :

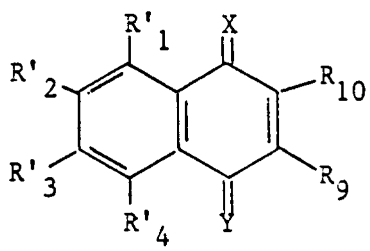

(V)

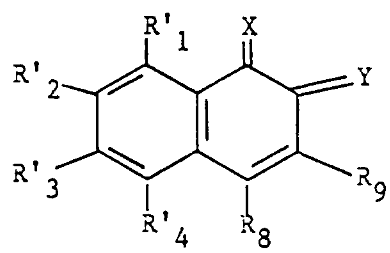

(VI)

dans lesquelles :

$R_8$, $R_9$ et $R_{10}$ ont les significations indiquées ci-dessus pour les composés de formules (II) et (II'); $R'_1$, $R'_2$, $R'_3$ et $R'_4$ ayant les significations préférés de $R_6$, $R_7$, $R_8$, $R_9$ et $R_{10}$;

X et Y ayant les mêmes significations que celles indiquées ci-dessus.

Les composés particulièrement préférés sont choisis parmi ceux dans lesquels $R'_1$, $R'_2$, $R'_3$, $R'_4$, $R_8$, $R_9$, $R_{10}$ désignent hydrogène, alkyle en $C_1$-$C_4$, alcoxy en $C_1$-$C_4$, halogène, acyl ($C_2$-$C_6$) amino ou $SO_3H$.

Parmi ces composés préférés, on peut citer :

- la 1,4-benzoquinone
- la 2-méthoxy 1,4-benzoquinone
- la 2-méthyl 1,4-benzoquinone
- la 2,6-diméthyl 1,4-benzoquinone
- la 2,3,5-trichloro 6-méthyl 1,4-benzoquinone
- la 2-acétylamino 1,4-benzoquinone
- la 2-acétylamino 3,5-diméthyl 1,4-benzoquinone
- la 2-chloro 1,4-benzoquinone
- la tétrachloro 1,2-benzoquinone
- la 2,3-diméthoxy 1,4-benzoquinone
- la 2-β-carboxyéthoxy 1,4-benzoquinone
- la 2-méthoxyméthyl 1,4-benzoquinone
- la 2-hydroxyméthyl 1,4-benzoquinone
- la 2-β-hydroxyéthylthio 1,4-benzoquinone
- la 2,5-bis-β-hydroxyéthylthio 1,4-benzoquinone
- la 2-β,γ-dihydroxypropylthio 1,4-benzoquinone
- la 2-β-carboxyéthylthio 1,4-benzoquinone
- la 2-carboxyméthyl 1,4-benzoquinone
- la 2-β-hydroxyéthylthio 6-méthyl 1,4-benzoquinone
- la 2-méthoxycarbonyl 3-méthoxy 1,4-benzoquinone
- la 2-méthoxycarbonyl 1,4-benzoquinone
- la 2-méthylthio 1,4-benzoquinone
- la 2-diméthylamino 1,4-benzoquinone
- la 2-acétylamino 5-méthoxy 1,4-benzoquinone
- la 2-(β-hydroxyéthylthio)méthyl 1,4-benzoquinone

- la 2-(méthylthio)méthyl 1,4-benzoquinone
- la 4,5-diméthoxy 1,2-benzoquinone
- la 4-méthyl 5-chloro 1,2-benzoquinone
- la 4,5-diméthyl 1,2-benzoquinone
- la 2,3-diméthyl 1,4-benzoquinone
- la 2-$\beta$-hydroxyéthoxy 1,4-benzoquinone
- la N-méthyl sulfonyl 1,4-benzoquinone monoimine
- la N-phényl sulfonyl 1,4-benzoquinone monoimine
- la 1,4-naphtoquinone
- la 1,2-naphtoquinone
- l'acide 1,2-naphtoquinone 4-sulfonique
- la 2,3-dichloro 1,4-naphtoquinone
- la N-2,6-trichloro 1,4-benzoquinone imine.

Dans les conditions habituelles d'une teinture, c'est-à-dire pour des temps de pose de 2 à 30 minutes et une température habituellement supportée par les modèles, par exemple entre 25° et 40°C, la concentration de l'aminoindole utilisé dans la composition (A) est de préférence comprise entre 0,01 et 0,3 mole/litre.

La concentration en dérivé quinonique est telle qu'elle permet l'oxydation de l'aminoindole dans les conditions habituelles utilisées par le coiffeur lors d'une teinture et elle est de préférence comprise entre 0,005 et 1 mole/litre dans la composition (B).

Le pH de la composition (A) est de préférence compris entre 2 et 10.

Le pH de la composition (B) est de préférence compris entre 2 et 10, celle-ci est cependant utilisée de préférence à un pH acide.

Les compositions (A) et (B) peuvent être conditionnées sous des formes habituellement utilisées, notamment dans la teinture des cheveux, en particulier sous forme de lotion plus ou moins épaissie, de gel, d'émulsion éventuellement conditionnée(s) en aérosol.

Le milieu approprié pour la teinture est généralement un milieu aqueux qui peut être constitué par l'eau ou un mélange d'eau et d'un solvant qui, lorsque la composition est appliquée sur les cheveux doit être cosmétiquement acceptable.

De tels solvants sont choisis plus particulièrement parmi les solvants organiques tels que les alcools inférieurs en $C_1$-$C_6$ comme l'alcool éthylique, l'alcool propylique ou isopropylique, l'alcool tertiobutylique, l'éthylèneglycol, le propylèneglycol, les éthers monométhylique, monoéthylique ou monobutylique de l'éthylèneglycol, l'acétate du monoéthyléther de l'éthylèneglycol, les monométhyléthers du propylèneglycol et du dipropylène glycol, le lactate de méthyle.

Les solvants particulièrement préférés sont l'alcool éthylique et le propylèneglycol.

Les solvants, lorsqu'ils sont utilisés, le sont plus particulièrement dans des concentrations comprises entre 10 et 50% pour les alcools inférieurs et pour les concentrations élevées en aminoindole.

Les compositions (A) et (B) conformes à l'invention peuvent également être stockées en milieu solvant anhydre. Les solvants sont choisis parmi les solvants définis ci-dessus. On appelle milieu anhydre un milieu contenant moins de 1% d'eau.

Les compositions conformes à l'invention, lorsqu'elles sont utilisées pour la teinture des cheveux, peuvent également contenir tous autres adjuvants habituellement utilisés en cosmétique et plus particulièrement des agents tensio-actifs anioniques, cationiques, non-ioniques, amphotères ou leurs mélanges, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les compositions (A) et/ou (B) utilisables dans le procédé conforme à l'invention, peuvent contenir d'autres colorants habituellement utilisés pour la teinture des fibres kératiniques et en particulier des colorants directs tels que les dérivés nitrés benzéniques, les colorants d'oxydation de type para ou ortho et des coupleurs ou des colorants d'oxydation dits "rapides", c'est-à-dire des molécules à structure benzénique, précurseurs de colorants susceptibles de générer les composés colorés par simple oxydation à l'air pendant le temps de pose sur la chevelure qui est généralement inférieur à 1 heure et en l'absence de tout autre agent oxydant.

Ces compositions peuvent également contenir des colorants quinoniques de la famille des benzoquinones, des benzoquinones imines ou diimines, des naphtoquinones, des naphtoquinone-imines ou naphtoquinone-di-imines, qui ne répondent pas aux conditions de potentiel définies ci-dessus. Ces colorants sont, dans ce cas, utilisés pour apporter leur propre nuance à la teinture.

En vue de la mise en oeuvre du procédé conforme à l'invention, on peut conditionner les différentes compositions dans un dispositif à plusieurs compartiments encore appelé "kit" ou nécessaire de teinture comportant tous les composants destinés à être appliqués pour une même teinture sur les fibres kératiniques et en particulier les cheveux, en applications successives avec ou sans prémélange.

De tels dispositifs peuvent comporter un premier compartiment contenant la composition (A) et un second compartiment comportant la composition (B). Une autre variante peut également consister à stocker la composition (A) et/ou la composition (B) dans un milieu solvant anhydre et à prévoir un troisième compartiment contenant un milieu aqueux approprié pour la teinture et cosmétiquement acceptable si les compositions sont destinées à être appliquées sur les cheveux. Dans ce cas, on mélange tout juste avant l'emploi le contenu du troisième compartiment dans l'un ou l'autre ou les deux compartiments contenant les compositions anhydres (A) et/ou (B).

Le procédé conforme à l'invention est mis en oeuvre de préférence en appliquant, dans un premier temps, la composition (A) et dans un second temps, la composition (B). Il peut être utilisé, notamment pour teindre les cheveux humains, naturels ou déjà teints, permanentés ou non, ou défrisés, les cheveux fortement ou légèrement décolorés et éventuellement permanentés.

Dans ce cas, on applique la composition (A) à une température qui peut être supportée par la tête, c'est-à-dire comprise entre 25 et 40 °C, pendant 2 à 30 minutes et on fait suivre avec ou sans rinçage intermédiaire cette application par l'application de la composition (B) contenant le dérivé quinonique qui est maintenu au contact des cheveux pendant 5 à 30 minutes, la température de teinture étant également comprise entre 25 et 40 °C.

Le procédé conforme à l'invention peut également être mis en oeuvre, en vue de nuancer ou rafraîchir une teinture qui aurait été effectuée à l'aide d'aminoindoles, en appliquant la composition (B) plusieurs heures ou jours après la teinture à l'aide d'aminoindoles.

Il est également possible d'utiliser ce procédé pour la teinture des fourrures ou de la laine dans les conditions habituelles industrielles de température, de temps de contact et de concentration.

Les exemples qui suivent servent à illustrer le procédé de teinture conforme à l'invention.

EXEMPLE DE PREPARATION 1

Préparation du 6-N-β-hydroxyéthylaminoindole

Etape 1

Préparation du 6-N-(β-chloroéthoxycarbonyl)aminoindole

On chauffe au reflux 0,05 mole (6,6 g) de 6-aminoindole, 5,5 g de carbonate de calcium dans 30 ml de dioxane. On ajoute peu à peu 0,055 mole (7,9 g) de chloroformiate de β-chloréthyle. Le mélange réactionnel est dilué à la glace. Le produit attendu précipite. Il fond à 134 °C.

L'analyse du produit recristallisé de l'éthanol donne les résultats suivants :

| Analyse pour $C_{11}H_{11}N_2O_2Cl$ | | | | | |
|---|---|---|---|---|---|
| | C | H | Cl | O | N |
| Calculé | 55,36 | 4,65 | 14,85 | 13,41 | 11,74 |
| Trouvé | 55,40 | 4,68 | 14,72 | 13,27 | 11,67 |

Etape 2

Préparation du 6-N-β-hydroxyéthylaminoindole

On ajoute 0,28 mole (66,5 g) de 6-N-(β-chloroéthoxycarbonyl)aminoindole à 200 ml de soude 4N et 66,5 ml d'éthanol. Le mélange réactionnel est chauffé 1 heure au reflux. On précipite le produit attendu par ajout de glace. Il fond à 99 °C.

L'analyse élémentaire du produit obtenu donne les résultats suivants :

| Analyse pour $C_{10}H_{12}N_2O$ | | | | |
|---|---|---|---|---|
| | C | H | N | O |
| Calculé | 68,16 | 6,86 | 15,90 | 9,08 |
| Trouvé | 67,88 | 6,91 | 15,91 | 9,15 |

EXEMPLE DE PREPARATION 2

Préparation du 6-N-($\beta$,$\gamma$-dihydroxypropyl)amino indole

On dissout 26,4 g de 6-amino indole dans 70 ml d'alcool absolu. On ajoute 29,6 g de glycidol et agite 4 heures à 30-40°C. On verse sur 200 g d'eau glacée et on extrait par trois fois 100 ml d'acétate d'éthyle. On lave le solvant à l'eau. On le sèche sur $Na_2$ $SO_4$ et chasse à sec sous vide.

Le résidu huileux est repris trois fois dans 0,6 litre d'éther isopropylique au reflux. On filtre l'éther, chasse à sec sous vide; on reprend l'huile résiduelle dans 10cc d'acétate d'éthyle et on purifie sur colonne de silice (éluant acétate d'éthyle 9/Heptane 1).

La fraction contenant le produit attendu est évaporé à sec sous vide.

EXEMPLE DE PREPRARATION 3

SYNTHESE DU 4-METHYL 6-AMINOINDOLE

1) SYNTHESE DE LA 2,3-DIMETHYL 5-NITROANILINE

On coule 363 g de 2,3-xylidine dans 1,8 litre d'acide sulfurique pur en maintenant la température à 40°C. A cette solution refroidie à 12°C, on ajoute un mélange sulfonitrique (132 ml d'acide nitrique (d=1,52) et 180 ml d'acide sulfurique pur) goutte à goutte en 1 heure, la température ne dépassant pas 15°C. Après 15 minutes, on verse sur 6 kg de glace sous agitation.

On essore le précipité beige de sulfate, lave deux fois avec 0,5 litre d'eau, puis avec trois fois 0,5 litre d'acétone. Le précipité est empâté avec 0,5 litre d'acétone, alcalinisé avec de l'ammoniaque puis dilué avec 1,5 litre d'eau glacée. Le précipité jaune est essoré, lavé à l'eau puis séché. On obtient un solide jaune possédant les caractéristiques suivantes :

| Analyse élémentaire pour $C_8H_{10}N_2O_2$ | | | | |
|---|---|---|---|---|
| | C | H | N | O |
| Calculé | 57,83 | 6,02 | 16,87 | 19,28 |
| Trouvé | 57,91 | 6,03 | 16,78 | 19,20 |

2) SYNTHESE DU N-(2,3-DIMETHYL 5-NITROPHEHYLE)

FORMIMIDATE DE METHYLE

On mélange 66,4 g de 2,3-diméthyl 5-nitroaniline, 0,4 litre de triméthylorthoformiate et 1,6 g d'acide paratoluènesulfonique et on porte 3 heures au reflux.

On verse sur 1 kg de glace, on essorte le précipité puis on lave avec deux fois 0,5 litre d'eau. Le précipité est redissous dans 0,2 litre d'acétate d'éthyle et filtré à chaud. Le solide obtenu après refroidissement est essoré, lavé à l'éther de pétrole et séché.

La cristallisation de l'éther isopropylique conduit à des cristaux blancs possédant les caractéristiques suivantes :

Point de fusion : 108°C

| Analyse élémentaire pour $C_{10}H_{12}N_2O_3$ | | | | |
|---|---|---|---|---|
| | C | H | N | O |
| Calculé<br>Trouvé | 57,69<br>57,74 | 5,81<br>5,84 | 13,45<br>13,39 | 23,05<br>23,25 |

3) SYNTHESE DU 4-METHYL 6-NITROINDOLE

A une solution de N-(2,3-diméthyl 5-nitrophényl)formimidate de méthyle (52 g) dans 0,37 litre de diméthylformamide, on ajoute une solution de 31 g d'éthoxylate de potassium, 51 ml d'oxalate d'éthyle et 0,25 litre de diméthylformamide.On porte la température du mélange à 40°C pendant 3 heures. Le précipité est essoré, lavé à l'eau. On reprend le précipité dans l'éther isopropylique à chaud puis on filtre.

Le filtrat est évaporé puis soumis à une chromatographie sur silice (éluant : heptane/acétate d'éthyle 9/1). On obtient un solide jaune possédant les caractéristiques suivantes :

Point de fusion : 145°C

| Analyse élémentaire pour $C_9H_8N_2O_2$ | | | | |
|---|---|---|---|---|
| | C | H | N | O |
| Calculé<br>Trouvé | 61,36<br>61,30 | 4,58<br>4,58 | 15,90<br>15,88 | 18,16<br>18,20 |

4) SYNTHESE DU 4-METHYL 6-AMINOINDOLE

On mélange 4,3 g de dérivé nitré préparé à l'étape 3, 20 ml d'éthanol, 9 ml de cyclohexène, 3 ml d'eau et 2,2 g de palladium sur charbon à 10% puis on porte 2 heures au reflux.

On filtre à chaud, lave le catalyseur avec de l'alcool, évapore le filtrat sous vide. Le précipité est repris dans l'éther isopropylique, traité avec du charbon végétal, puis filtré sur célite.

Après évaporation du filtrat, on obtient un solide beige possédant les caractéristiques suivantes :

Point de fusion : 102°C.

| Analyse élémentaire pour $C_8H_{10}N_2$ | | | |
|---|---|---|---|
| | C | H | N |
| Calculé<br>Trouvé | 73,97<br>73,84 | 6,85<br>6,99 | 19,18<br>19,06 |

EXEMPLE DE PREPARATION 4

SYNTHESE DU 4-AMINO 7-METHYLINDOLE

On utilise le même procédé de réduction du point 4 de l'exemple 3, en utilisant le 4-nitro 7-méthylindole à la place du 4-méthyl 6-nitroindole.

On obtient un solide jaune pâle possédant les caractéristiques suivantes :

Point de fusion : 131°C

| Analyse élémentaire pour $C_8H_{10}N_2$ | | | |
|---|---|---|---|
| | C | H | N |
| Calculé | 73,97 | 6,85 | 19,18 |
| Trouvé | 73,95 | 6,94 | 19,11 |

EXEMPLE DE PREPARATION 5

SYNTHESE DU 3-METHYL 6-AMINO 7-ETHYLINDOLE

On porte à 90°C un mélange de 0,5 litre d'éthanol à 96°, 0,5 litre d'acide acétique glacial et 100 g de fer pur réduit à l'hydrogène. On ajoute par portion 51 g de 3-méthyl 6-nitro 7-éthylindole en 15 minutes.

Après 2 heures à 95°C, on filtre les boues ferriques, le filtrat est refroidi et dilué par trois volumes d'eau. On extrait avec trois fois 0,5 litre d'acétate d'éthyle, les phases organiques sont lavées, séchées et évaporées. Le résidu est repris dans l'acétate d'éthyle, traité avec du charbon végétal, filtré et refroidi.

Le précipité beige clair est essoré, lavé à l'acétate d'éthyle. Le solide est dissous dans 0,1 litre d'eau, alcalinisé avec de l'ammoniaque, une huile précipité puis se solidifie. Le précipité est filtré puis lavé jusqu'à la neutralité puis séché. On obtient un solide blanc possédant les caractéristiques suivantes :

Point de fusion : 106°C.

| Analyse élémentaire pour $C_{11}H_{14}N_2$ | | | |
|---|---|---|---|
| | C | H | N |
| Calculé | 75,82 | 8,10 | 16,08 |
| Trouvé | 76,01 | 8,10 | 16,06 |

EXEMPLES DE PROCEDES DE TEINTURE

EXEMPLE 1

On imprègne une mèche de 1 g de cheveux naturels à 90% de blancs par 5 ml d'une solution de 6-aminoindole à 2,5% en milieu hydroéthanolique (80/20) pendant 15 minutes.

On rince la mèche à l'eau courante et on l'essore. On imprègne à nouveau cette mèche par 5 ml d'une solution à 2% de 1,4-benzoquinone en milieu hydroéthanolique (50/50) pendant 8 minutes.

On rince la mèche à l'eau courante et on effectue un shampooing avec une solution aqueuse à 5% de lauryl sulfate de sodium.

Après rinçage et séchage on obtient une mèche colorée en noir.

Le potentiel d'oxydoréduction du 6-aminoindole déterminé en milieu phosphate à pH 7 sur électrode de carbone vitreux par voltamétrie est $E_i$ = 320 mV.

Le potentiel d'oxydoréduction de la 1,4-benzoquinone déterminé en milieu phosphate à pH 7 par polarographie sur électrode de mercure par rapport à l'électrode au calomel est $E_q$ = 10 mV. $\Delta E$ = 310 mV.

EXEMPLE 2

On applique le même procédé qu'à l'exemple 1 mais on utilise une solution à 2,5% de 5-aminoindole dans un mélange hydroéthanolique (80/20) à la place du 6-aminoindole.

On obtient une mèche colorée en châtain.

Les valeurs des potentiels d'oxydoréduction déterminées comme dans l'exemple 1 sont : $E_i$ = 390 mV, $E_q$ = 10 mV, $\Delta E$ = 380 mV.

EXEMPLE 3

On applique le même procédé que dans l'exemple 1 mais on utilise une solution à 2,5% de 7-aminoindole en solution dans un mélange hydroéthanolique (80/20) à la place du 6-aminoindole.

On obtient une mèche colorée en gris foncé.

Les valeurs des potentiels d'oxydoréduction déterminées comme dans l'exemple 1 sont : $E_i$ = 420 mV, $E_q$ = 10 mV. $\Delta E$ = 410 mV.

EXEMPLE 4

On applique le même procédé que dans l'exemple 1 en utilisant une solution à 2,5% de 6-N-$\beta$-hydroxyéthyl aminoindole préparé selon l'exemple de préparation 1, dans un mélange hydroéthanolique (80/20) à la place du 6-aminoindole.

On obtient une mèche colorée en noir.

Les valeurs des potentiels d'oxydoréduction déterminées comme dans l'exemple 1 sont : $E_i$ = 265 mV, $E_q$ = 10 mV. $\Delta E$ = 255 mV.

EXEMPLE 5

On imprègne une mèche de 1 g de cheveux naturels à 90% de blancs par 5 ml d'une solution de 4-aminoindole à 2,5% en milieu hydroéthanolique (80/20) pendant 15 minutes.

On rince la mèche à l'eau courante et on l'essore.

On imprègne à nouveau cette mèche par 5 ml d'une solution à 2% de N-2,6-trichloro 1,4-benzoquinone imine en milieu hydroéthanolique (50/50) pendant 10 minutes.

Après avoir rincé, lavé, rincé une nouvelle fois, puis séché, on obtient une mèche colorée en bleu foncé.

Les valeurs des potentiels d'oxydoréduction déterminées comme dans l'exemple 1 sont : $E_i$ = 365 mV, $E_q \geqq$ à 180 mV. $\Delta E \leqq$ à 185 mV.

EXEMPLE 6

On applique le même procédé que dans l'exemple 5 en utilisant une solution à 2,5% de 7-aminoindole dans un mélange hydroéthanolique (80/20) à la place du 4-aminoindole.

On obtient une mèche colorée en bleu-vert.

Les valeurs des potentiels d'oxydo-réduction déterminés comme dans l'exemple 1 sont :

$E_i$ = 420 mV, $E_q \geqq$ à 180, mV $\Delta E \leqq$ à 240 mV

EXEMPLE 7

On applique le même procédé que dans l'exemple 1 en utilisant une suspension à 2% de 1,2-naphtoquinone en milieu hydroéthanolique 50/50 à la place de la 1,4-benzoquinone.

On obtient une mèche colorée en violine.

Les valeurs des potentiels d'oxydo-réduction déterminés comme dans l'exemple 1 sont :

$E_i$ = 320 mV, $E_q$ = -55 mV, $\Delta E$ = 375 mV

EXEMPLE 8

On imprègne une mèche de 1 g de cheveux naturels à 90% de blancs par 5 ml d'une solution de 2,3-diméthyl 6-aminoindole à 2,5% en milieu hydroalcoolique (80/20) pendant 15 minutes.

On rince la mèche à l'eau courant et on l'essore. On imprègne à nouveau cette mèche par 5 ml d'une suspension à 2% de 1,2-naphtoquinone en milieu hydroéthanolique 50/50 pendant 8 minutes.

On rince à l'eau courant et on effectue un shampooing avec une solution aqueuse à 5% de laurylsulfate de sodium.

Après rinçage et séchage on obtient une mèche colorée en gris foncé avec un reflet violine.

Les potentiels d'oxydo-réduction déterminés comme dans l'exemple 1 sont :

$E_i$ = 145 mV, $E_q$ = -55 mV, $\Delta E$ = 200 mV

EXEMPLE 9

On applique le même procédé que dans l'exemple 1, mais on révèle en couleur par la 2-méthyl 1,4-benzoquinone (toluquinone) dissoute à raison de 2% dans une solution hydroalcoolique 50/50.

On obtient une mèche colorée en châtain moyen.

Les valeurs des potentiels d'oxydo-réduction déterminées comme dans l'exemple 1 sont :

$E_i$ = 320 mV, $E_q$ = -45 mV, $\Delta E$ = 365 mV

EXEMPLE 10

On applique le même procédé que dans l'exemple 1, mais on révèle la couleur par la 2-$\beta$-hydroxyéthylthio 1,4-benzoquinone dissoute à raison de 2% dans une solution hydroalcoolique 50/50.

On obtient une mèche colorée en vert.

Les valeurs des potentiels d'oxydo-réduction déterminées comme dans l'exemple 1 sont :

$E_i$ = 320 mV, $E_q$ = 25 mV, $\Delta E$ = 295 mV

EXEMPLE 11

On applique le même procédé que dans l'exemple 8 mais on révèle la couleur par la 2-$\beta$-hydroxyéthylthio 1,4-benzoquinone dissoute à raison de 2% dans une solution hydroalcoolique 50/50 en remplacement de la 1,2-naphtoquinone en suspension.

On obtient une mèche gris foncé.

Les valeurs des potentiels d'oxydo-réduction déterminées comme dans l'exmeple 1 sont :

$E_i$ = 145 mV, $E_q$ = 25 mV, $\Delta E$ = 120 mV

EXEMPLE 12

On applique le même procédé que dans l'exemple 1, mais on révèle la couleur par la 2-hydroxyméthyl 1,4-benzoquinone à 2% dans une solution hydroéthanolique 50/50 en remplacement de la 1,4-benzoquinone.

On obtient une coloration brune.

Les valeurs des potentiels d'oxydo-réduction déterminées comme dans l'exemple 1 sont :

$E_i$ = 320 mV, $E_q$ = 5 mV, $\Delta E$ = 315 mV

EXEMPLE 13

On applique le même procédé que dans l'exemple 1, mais on remplace le 6-aminoindole par le 2,3-diméthyl 5-éthyl 6-aminoindole.

On obtient ainsi, après révélation, avec la 1,4-benzoquinone une coloration rouge cuivré.

Les valeurs des potentiels d'oxydo-réduction déterminées comme dans l'exemple 1 sont :

$E_i$ = 120 mV, $E_q$ = 10 mV, $\Delta E$ = 110 mV

EXEMPLE 14

On applique le même procédé que dans l'exemple 1, mais on remplace le 6-aminoindole par le 2,3-diméthyl 4-méthyl 6-aminoindole.

On obtient ainsi après révélation une coloration brun-roux.

Les valeurs des potentiels d'oxydo-réduction déterminées comme dans l'exemple 1 sont :

$E_i$ = 90 mV, $E_q$ = 10 mV, $\Delta E$ = 80 mv

## Revendications

1. Procédé de teinture des fibres kératiniques caractérisé par le fait que l'on applique sur ces fibres au moins une composition(A) contenant dans un milieu approprié pour la teinture au moins un aminoindole, différent du 2,3-diméthyl 5-hydroxy 6-amino indole et du 2,3-diméthyl 6-hydroxy 5-aminoindole, l'application de la composition (A) étant précédée ou suivie par l'application d'une composition (B) contenant, dans un milieu approprié pour la teinture, au moins un dérivé quinonique choisi parmi les ortho- ou para-benzoquinones, les ortho- ou para-benzoquinones monoimines ou diimines, les 1,2- ou 1,4-naphtoquinones, les ortho- ou para-benzoquinones sulfonimides, les $\alpha,\omega$-alkylène bis-1,4-benzoquinones ou les 1,2- ou 1,4-naphtoquinones monoimines ou diimines, les aminoindoles et les dérivés quinoniques étant choisis de façon à ce que la différence de potentiel d'oxydoréduction $\Delta E$ entre le potentiel d'oxydoréduction $E_i$ des aminoindoles déterminé à pH 7 en milieu phosphate sur électrode de carbone vitreux par voltamétrie, et le potentiel d'oxydoréduction $E_q$ du dérivé quinonique déterminé à pH 7 en milieu phosphate par polarographie sur électrode de mercure par rapport à l'électrode au calomel saturé soit telle que :

   $\Delta E = E_i - E_q \leqq 470$ mV.

2. Procédé selon la revendication 1, caractérisé par le fait que les aminoindoles sont choisis parmi les composés répondant à la formule :

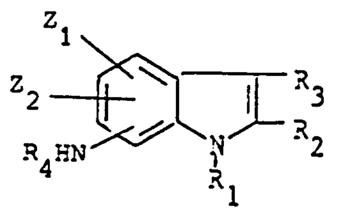

(I)

   dans laquelle :

   $R_1$ désigne un atome d'hydrogène ou un groupement alkyle en $C_1$-$C_4$ linéaire ou ramifié;

   $R_2$ et $R_3$ désignent un atome d'hydrogène, un groupement alkyle en $C_1$-$C_4$, un groupement carboxyle ou un groupement alcoxy ($C_1$-$C_4$) carbonyle;

   $R_4$ désigne un atome d'hydrogène ou un groupement alkyle en $C_1$-$C_4$ ou hydroxyalkyle en $C_1$-$C_4$ ou polyhydroxyalkyle en $C_2$-$C_4$, acétyle, amino- alkyle en $C_1$-$C_6$ dont l'amine peut être éventuellement mono ou disubstituée par un alkyle en $C_1$-$C_4$; à l'exception du 2,3-diméthyl 5-hydroxy 6-aminoindole et du 2,3-diméthyl 6-hydroxy 5-aminoindole.

   $Z_1$ et $Z_2$, identiques ou différents, représentent un atome d'hydrogène, un groupement alkyle en $C_1$-$C_4$, un hydroxy, un halogène ou un alcoxy en $C_1$-$C_4$;

   $NHR_4$ occupant les positions 4, 5, 6 ou 7; et leurs sels.

3. Procédé selon la revendication 1 ou 2 caractérisé par le fait que les aminoindoles sont choisis parmi : le 5-aminoindole, le 6-aminoindole, le 7-aminoindole, le 6-N-$\beta$-hydroxyéthylaminoindole, le 6-N-$\beta$-hydroxyéthylamino 1-méthylindole, le 6-méthyl aminoindole, le 6-amino N-méthylindole, le 6-amino 2-carboxy-indole, le 4-amino 2,3-diméthylindole, le 6-amino 2,3-diméthylindole, le 7-amino 2,3-diméthylindole, le 6-amino 3-éthyl 2-méthylindole, le 7-amino 3-éthyl 2-méthylindole, le 6-amino 3-méthylindole, le 6-amino 2-méthylindole et le 6-amino 2-éthoxycarbonylindole, le 4-aminoindole, le 5-amino 6-méthoxy 2,3-diméthylindole, le 6-amino 5-méthoxy 2,3-diméthylindole,
   le 5-amino N-méthylindole, le 6-N-($\beta,\gamma$-dihydroxypropyl)aminoindole, le 6-amino 2,3,4, 5-tétraméthylindole, le 6-amino 5-chloro 2,3-diméthylindole, le 6-amino 5-éthyl 2,3-diméthylindole, le 6-amino 2,3,4-triméthylindole,le 6-amino 5-hydroxy 2-méthylindole, le 4-méthylaminoindole, le 4-amino N-méthylindole, le 6-amino 2,3,7-triméthylindole, le 6-amino 2,3,5-triméthylindole, le 5-acétylamino 6-méthoxy 2,3-diméthylindole, le 6-amino 4-méthylindole, le 6-amino 5-méthylindole, le 4-amino 7-méthylindole, le 6-amino 7-éthyl 3-méthylindole, le 6-amino 5,7-diméthylindole, le 6-amino 5,7-diéthylindole, le 7-amino 5-méthyl 2-éthoxycarbonylindole, le 7-amino 5-chloro 2-éthoxycarbonylindole, le 7-amino 5-éthoxy 2-éthoxycarbonylindole, le 7-amino 5-méthoxy 2-éthoxycarbonylindole, le 7-(4'-diméthylamino 1'-méthylbutyl)amino 5-méthoxyindole, le 7-(4'-diméthylaminobutyl)amino 5-méthoxyindole, le 6-amino 5-fluoroindole, le 6-amino 5-fluoro 1-sec.butylindole, le 6-amino 5-fluoro 1-n-propylindole, le 6-amino 2-méthoxy

carbonyl 5-méthoxy N-méthylindole, le 6-amino 5-méthoxy 2-méthoxycarbonylindole, le 6-amino 5-méthoxy 2-éthoxycarbonylindole, le 6-amino 5-méthoxy 2-carboxyindole, le 6-amino 5-hydroxy 1,2-diméthylindole, le 6-amino 4-méthoxy 2-méthoxycarbonylindole.

**4.** Procédé selon l'une quelconque des revendications 1 à 3, caractérisé par le fait que les dérivés quinoniques sont choisis parmi les composés répondant aux formules (II) et (II')) :

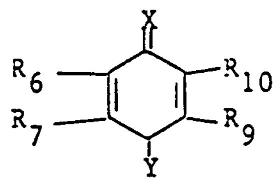

(II)

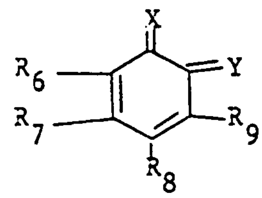

(II')

dans lesquelles :

X désigne oxygène ou un groupement $NR_{11}$;

Y désigne oxygène ou un groupement $NR_{12}$;

$R_{11}$ et $R_{12}$, identiques ou différents, désignant hydrogène, halogène, un radical alkyle en $C_1$-$C_4$, hydroxyalkyle en $C_1$-$C_4$, un radical alkyl ($C_1$-$C_4$) sulfonyle ou un radical phénylsulfonyle;

$R_6$, $R_7$, $R_8$, $R_9$ et $R_{10}$ désignant, indépendamment l'un de l'autre, hydrogène, un radical alkyl ($C_1$-$C_4$), un groupement carboxyle, alkyl ($C_1$-$C_4$) carbonyle, alcoxy ($C_1$-$C_4$) carbonyle, alcoxy ($C_1$-$C_4$) méthyle, alkyl ($C_1$-$C_4$) thiométhyle, hydroxyalkyl ($C_1$-$C_4$) thiométhyle, hydroxyalkyl ($C_1$-$C_4$) sulfinyle,

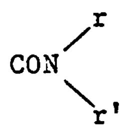

(r et r' désignant, indépendamment l'un de l'autre,
hydrogène ou alkyle en $C_1$-$C_4$), carboxyalkyle en $C_1$-$C_4$, halogène, hydroxyalkyl ($C_1$-$C_4$), amino substitué ou non par un ou deux groupes alkyle ou hydroxyalkyle en $C_1$-$C_4$, acyl ($C_2$-$C_6$)amino, $SO_3M$, où M désigne hydrogène, K ou Na, un groupement sulfoxyde, sulfone ou sulfonamide, éventuellement substitué, ou bien un radical $OZ_1$ dans lequel $Z_1$ peut être hydrogène, alkyl ($C_1$-$C_4$), hydroxyalkyl ($C_1$-$C_4$), carboxyalkyl ($C_1$-$C_4$), phényle éventuellement substitué par alcoxy en $C_1$-$C_4$, ou encore un radical $-SZ_2$ dans lequel $Z_2$ est un groupe alkyle en $C_1$-$C_4$, hydroxyalkyle en $C_1$-$C_4$, dihydroxyalkyle en $C_2$-$C_4$, carboxyalkyle en $C_1$-$C_4$;

$R_6$ et $R_7$ pouvant former avec les atomes de carbone auxquels ils sont rattachés, le groupement cyclique suivant :

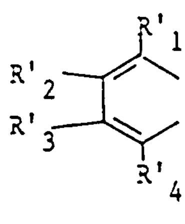

(III)

dans lequel :

$R'_1$, $R'_2$, $R'_3$ et $R'_4$ ont les significations indiquées ci-dessus pour $R_6$, $R_7$, $R_8$, $R_9$, $R_{10}$, lorsqu'ils ne forment pas de cycles;

**5.** Procédé selon l'une quelconque des revendications 1 à 4, caractérisé par le fait que les dérivés quinoniques sont choisis parmi les benzoquinones de formules :

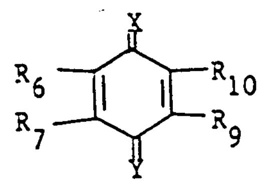

(II)

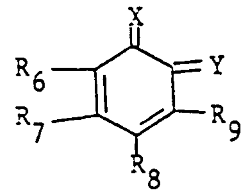

(II')

dans lesquelles :

$R_6$, $R_7$, $R_8$, $R_9$, $R_{10}$ désignent, indépendamment l'un de l'autre, hydrogène, alkyle inférieur en $C_1$-$C_4$, alcoxy inférieur en $C_1$-$C_4$, halogène, acylamino en $C_2$-$C_6$, $SO_3M$, alcoxy ($C_1$-$C_4$) méthyle, carboxy alkyl ($C_1$-$C_4$), alcoxy ($C_1$-$C_4$) carbonyle, dialkyl ($C_1$-$C_4$) amino, $OZ_1$ dans lequel $Z_1$ représente carboxylalkyl ($C_1$-$C_4$), hydroxyalkyl ($C_1$-$C_4$), $SZ_2$ dans lequel $Z_2$ représente hydroxyalkyl ($C_1$-$C_4$)-,dihydroxyalkyl ($C_2$-$C_4$), carboxyalkyl ($C_1$-$C_4$), alkyl ($C_1$-$C_4$).

X désigne oxygène ou le groupe $NR_{11}$;

Y désigne oxygène ou le groupement $NR_{12}$;

$R_{11}$ et $R_{12}$, indépendamment l'un de l'autre, désignent hydrogène, halogène ou alkyle inférieur en $C_1$-$C_4$; méthylsulfonyle ou phénylsulfonyle.

**6.** Procédé selon l'une quelconque des revendications 1 à 5, caractérisé par le fait que les dérivés quinoniques sont choisis parmi les composés de formules :

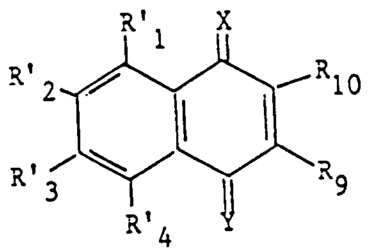

(V)

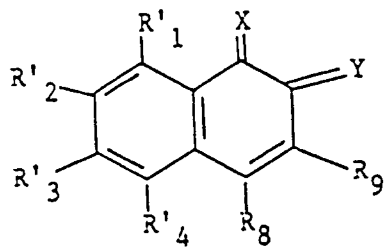

(VI)

dans lesquelles :

$R_8$, $R_9$ et $R_{10}$ ont les significations indiquées dans la revendication 5; $R'_1$, $R'_2$, $R'_3$, $R'_4$ ont les significations indiquées pour $R_6$, $R_7$, $R_8$, $R_9$ et $R_{10}$ dans la revendication 5.

X et Y ayant les mêmes singifications que celles indiquées dans la revendication 5.

**7.** Procédé selon l'une quelconque des revendications 1 à 5, caractérisé par le fait que les dérivés quinoniques sont choisis parmi les composés suivants : la 1,4-benzoquinone, la 2-méthoxy 1,4-benzoquinone, la 2-méthyl 1,4-benzoquinone, la 2,6-diméthyl 1,4-benzoquinone, la 2,3,5-trichloro 6-méthyl 1,4-benzoquinone, la 2-acétylamino 1,4-benzoquinone, la 2-acétylamino 3,5-diméthyl 1,4-benzoquinone, la 2-chloro 1,4-benzoquinone, la tétrachloro 1,2-benzoquinone, la 2,3-diméthoxy 1,4-benzoquinone, la 2-$\beta$-carboxyéthoxy 1,4-benzoquinone, la 2-méthoxyméthyl 1,4-benzoquinone, la 2 - hydroxyméthyl 1,4-benzoquinone, la 2-$\beta$-hydroxyéthylthio 1,4-benzoquinone, la 2,5-bis-$\beta$-hydroxyéthylthio 1,4-benzoquinone, la 2-$\beta$,$\gamma$-dihydroxypropylthio 1,4-benzoquinone, la 2-$\beta$-carboxyéthylthio 1,4-benzoquinone, la 2-carboxyméthyl 1,4-benzoquinone, la 2-$\beta$-hydroxyéthylthio 6-méthyl 1,4-benzoquinone, la 2-méthoxycarbonyl 3-méthoxy 1,4-benzoquinone, la 2-méthoxycarbonyl 1,4-benzoquinone, la 2-méthylthio 1,4-benzoquinone, la 2-diméthylamino 1,4-benzoquinone, la 2-acétylamino 5-méthoxy 1,4-benzoquinone, la 2-($\beta$-hydroxyéthylthio)méthyl 1,4-benzoquinone, la 2-(méthylthio)méthyl 1,4-benzoquinone, la 4,5-diméthoxy 1,2-benzoquinone, la 4-méthyl 5-chloro 1,2-benzoquinone, la 4,5-diméthyl 1,2-benzoquinone, la 2,3-diméthyl 1,4-benzoquinone, la 2-$\beta$-hydroxyéthoxy 1,4-benzoquinone, la N-méthyl sulfonyl 1,4-benzoquinone monoimine, la N-phényl sulfonyl 1,4-benzoquinone monoimine, la 1,4-naphtoquinone, la 1,2-naphtoquinone, l'acide 1,2-naphtoquinone 4-sulfonique, la 2,3-dichloro 1,4-naphtoquinone, la N-2,6-trichloro 1,4-benzoquinone imine.

**8.** Procédé selon l'une quelconque des revendications 1 à 7, caractérisé par le fait que l'aminoindole est présent dans la composition (A) en une concentration variant entre 0,01 et 0,3 mole/litre.

**9.** Procédé selon l'une quelconque des revendications 1 à 7, caractérisé par le fait que le dérivé quinonique est présent dans la composition (B) dans des concentrations comprises entre 0,005 et 1 mole/litre.

**10.** Procédé selon l'une quelconque des revendications 1 à 9, caractérisé par le fait que le pH des compositions (A) et (B) est indépendamment l'un de l'autre, compris entre 2 et 10.

**11.** Procédé selon la revendication 10, caractérisé par le fait que le pH de la composition (B) est acide.

**12.** Procédé selon l'une quelconque des revendications 1 à 11, caractérisé par le fait que les compositions (A) et (B) comprennent un milieu aqueux constitué par de l'eau ou un mélange d'eau et d'un solvant.

**13.** Procédé selon l'une quelconque des revendications 1 à 9, caractérisé par le fait que la composition (A) et/ou (B) sont constituées par un milieu solvant anhydre.

**14.** Procédé selon l'une quelconque des revendications 1 à 13, caractérisé par le fait que les compositions (A) et (B) contiennent indépendamment l'une de l'autre des agents tensio-actifs anioniques, cationiques, non-ioniques, amphotères ou leurs mélanges, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

**15.** Procédé selon l'une quelconque des revendications 1 à 14, caractérisé par le fait que les compositions (A) et/ou (B) contiennent d'autres colorants choisis parmi les colorants directs, les colorants d'oxydation, les coupleurs ou les colorants d'oxydation dits "rapides".

**16.** Procédé selon l'une quelconque des revendications 1 à 15, caractérisé par le fait que la composition (A) et/ou (B) contient également des colorants quinoniques de la famille des benzoquinones, des benzoquinones imines ou diimines, des naphtoquinones, des naphtoquinonimines, des naphtoquinonediimines dont les potentiels sont tels que $\Delta E$ soit supérieur à 470 mV.

**17.** Dispositif à plusieurs compartiments ou kit de teinture, caractérisé par le fait qu'il comprend dans un premier compartiment la composition (A) telle que définie dans l'une quelconque des revendications 1 à 16, et dans un second compartiment une composition (B) telle que définie dans l'une quelconque des revendications 1 à 16.

**18.** Dispositif à plusieurs compartiments ou kit de teinture caractérisé par le fait qu'il comporte dans un premier compartiment une composition (A) telle que définie dans l'une quelconque des revendications 1 à 16, dans un second compartiment la composition (B) telle que définie dans l'une quelconque des revendications 1 à 16, l'une au moins des deux compositions (A) et (B) comportant un milieu solvant anhydre et un troisième compartiment contenant un milieu aqueux pour la teinture et destiné à être mélangé tout juste avant l'emploi avec le contenu de l'un ou l'autre des deux compartiments contenant les compositions anhydres (A) et/ou (B).

**Claims**

**1.** Method for dyeing keratinous fibres, characterised in that at least one composition (A) containing at least one aminoindole different from 2,3-dimethyl-5-hydroxy-6-aminoindole and from 2,3-dimethyl-6-hydroxy-5-aminoindole in a medium appropriate for dyeing is applied to these fibres, the application of the composition (A) being preceded or followed by the application of a composition (B) containing, in a medium appropriate for dyeing, at least one quinone derivative chosen from ortho- or para-benzoquinones, ortho- or para-benzoquinone monoimines or diimines, 1,2- or 1,4-naphthoquinones, ortho-or para-benzoquinone sulphonimides, $\alpha,\omega$-alkylene-bis-1,4-benzoquinones, or 1,2- or 1,4-naphthoquinone monoimines or diimines, the aminoindoles and the quinone derivatives being chosen such that the difference in redox potential $\Delta E$ between the redox potential $E_i$ of the aminoindoles, determined at pH 7

in a phosphate medium on a vitreous carbon electrode by voltametry, and the redox potential $E_q$ of the quinone derivative, determined at pH 7 in a phosphate medium by polarography on a mercury electrode relative to the saturated calomel electrode, is such that

$\Delta E = E_i - E_q \leq 470$ mV.

**2.** Method according to Claim 1, characterised in that the aminoindoles are chosen from the compounds corresponding to the formula:

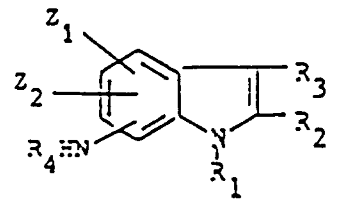

(I)

in which:

$R_1$ denotes a hydrogen atom or a straight-chain or branched $C_1$-$C_4$ alkyl group;

$R_2$ and $R_3$ denote a hydrogen atom, a $C_1$-$C_4$ alkyl group, a carboxyl group or a ($C_1$-$C_4$) alkoxycarbonyl group;

$R_4$ denotes a hydrogen atom or a $C_1$-$C_4$ alkyl or $C_1$-$C_4$ hydroxyalkyl or $C_2$-$C_4$ polyhydroxyalkyl or acetyl group or a $C_1$-$C_6$ aminoalkyl group in which the amine may optionally be monosubstituted or disubstituted by a $C_1$-$C_4$ alkyl; with the exception of 2,3-dimethyl-5-hydroxy-6-aminoindole and 2,3-dimethyl-6-hydroxy-5-aminoindole.

$Z_1$ and $Z_2$, which may be identical or different, represent a hydrogen atom, a $C_1$-$C_4$ alkyl group, a hydroxyl, a halogen or a $C_1$-$C_4$ alkoxy;

$NHR_4$ occupying the 4, 5, 6 or 7 positions; and their salts.

**3.** Method according to Claim 1 or 2, characterised in that the aminoindoles are chosen from:
5-aminoindole, 6-aminoindole, 7-aminoindole, 6-N-$\beta$-hydroxyethylaminoindole, 6-N-$\beta$-hydroxyethylamino-1-methylindole, 6-methylaminoindole, 6-amino-N-methylindole, 6-amino-2-carboxyindole, 4-amino-2,3-dimethylindole, 6-amino-2,3-dimethylindole, 7-amino-2,3-dimethylindole, 6-amino-3-ethyl-2-methylindole, 7-amino-3-ethyl-2-methylindole, 6-amino-3-methylindole, 6-amino-2-methylindole and 6-amino-2-ethoxycarbonylindole, 4-aminoindole, 5-amino-6-methoxy-2,3-dimethylindole, 6-amino-5-methoxy-2,3-dimethylindole, 5-amino-N-methylindole, 6-N-($\beta$,$\gamma$-dihydroxypropyl)aminoindole, 6-amino-2,3,4,5-tetramethylindole, 6-amino-5-chloro-2,3-dimethylindole, 6-amino-5-ethyl-2,3-dimethylindole, 6-amino-2,3,4-trimethylindole, 6-amino-5-hydroxy-2-methylindole, 4-methylaminoindole, 4-amino-N-methylindole, 6-amino-2,3,7-trimethylindole, 6-amino-2,3,5-trimethylindole, 5-acetylamino-6-methoxy-2,3-dimethylindole, 6-amino-4methylindole, 6-amino-5-methylindole, 4-amino-7-methylindole, 6-amino-7-ethyl-3-methylindole, 6-amino-5,7-dimethylindole, 6-amino-5,7-diethylindole, 7-amino-5-methyl-2-ethoxycarbonylindole, 7-amino-5-chloro-2-ethoxycarbonylindole, 7-amino-5-ethoxy-2-ethoxycarbonylindole, 7-amino-5-methoxy-2-ethoxycarbonylindole, 7-(4'-dimethylamino-1'-methylbutyl)amino-5-methoxyindole, 7-(4'-dimethylaminobutyl)amino-5-methoxyindole, 6-amino-5-fluoroindole, 6-amino-5-fluoro-1-sec-butylindole, 6-amino-5-fluoro-1-n-propylindole, 6-amino-2-methoxycarbonyl-5-methoxy-N-methylindole, 6-amino-5-methoxy-2-methoxycarbonylindole, 6-amino-5-methoxy-2-ethoxycarbonylindole, 6-amino-5-methoxy-2-carboxyindole, 6-amino-5-hydroxy-1,2-dimethylindole and 6-amino-4-methoxy-2-methoxycarbonylindole.

**4.** Method according to any one of Claims 1 to 3, characterised in that the quinone derivatives are chosen from the compounds corresponding to the formulae (II) and (II')) [sic]:

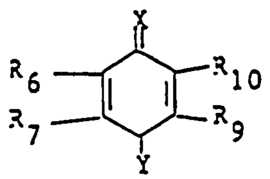

(II)

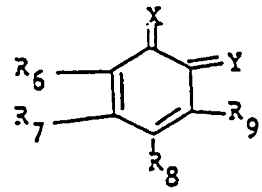

(II')

in which formulae:

X denotes oxygen or an $NR_{11}$ group;

Y denotes oxygen or an $NR_{12}$ group;

$R_{11}$ and $R_{12}$, which may be identical or different, denoting hydrogen, halogen, a $C_1$-$C_4$ alkyl or $C_1$-$C_4$ hydroxyalkyl radical, a ($C_1$-$C_4$) alkylsulphonyl radical or a phenylsulphonyl radical; and

$R_6$, $R_7$, $R_8$, $R_9$ and $R_{10}$ denote, independently of one another, hydrogen, a ($C_1$-$C_4$) alkyl radical, a carboxyl, ($C_1$-$C_4$) alkylcarbonyl, ($C_1$-$C_4$) alkoxycarbonyl, ($C_1$-$C_4$) alkoxymethyl, ($C_1$-$C_4$) alkylthiomethyl, ($C_1$-$C_4$) hydroxyalkylthiomethyl or ($C_1$-$C_4$) hydroxyalkylsulphinyl group,

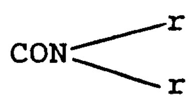

(r and r' denoting, independently of one another, hydrogen or $C_1$-$C_4$ alkyl), $C_1$-$C_4$ carboxyalkyl, halogen, ($C_1$-$C_4$) hydroxyalkyl, amino which is unsubstituted or substituted by one or two $C_1$-$C_4$ alkyl or hydroxyalkyl, ($C_2$-$C_6$) acylamino or $SO_3M$ groups, where M denotes hydrogen, K or Na, an optionally substituted sulphoxide, sulphone or sulphonamide group, or a radical $OZ_1$ in which $Z_1$ may be hydrogen, ($C_1$-$C_4$) alkyl, ($C_1$-$C_4$) hydroxyalkyl, ($C_1$-$C_4$) carboxyalkyl, or phenyl which is optionally substituted by $C_1$-$C_4$ alkoxy, or a radical $-SZ_2$ in which $Z_2$ is a $C_1$-$C_4$ alkyl, $C_1$-$C_4$ hydroxyalkyl, $C_2$-$C_4$ dihydroxyalkyl or $C_1$-$C_4$ carboxyalkyl group;

it being possible for $R_6$ and $R_7$ to form, with the carbon atoms to which they are attached, the following cyclic group:

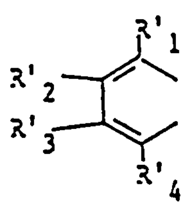

(III)

in which:

$R'_1$, $R'_2$, $R'_3$ and $R'_4$ have the meanings indicated above for $R_6$, $R_7$, $R_8$, $R_9$ and $R_{10}$ when they do not form rings;

**5.** Method according to any one of Claims 1 to 4, characterised in that the quinone derivatives are chosen from the benzoquinones of formulae:

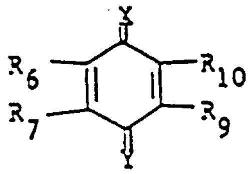

(II)

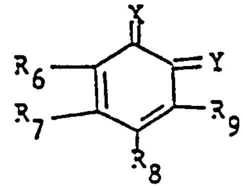

(II')

in which formulae:

$R_6$, $R_7$, $R_8$, $R_9$ and $R_{10}$ denote, independently of one another, hydrogen, $C_1$-$C_4$ lower alkyl, $C_1$-$C_4$ lower alkoxy, halogen, $C_2$-$C_6$ acylamino, $SO_3M$, ($C_1$-$C_4$) alkoxymethyl, ($C_1$-$C_4$) carboxyalkyl, ($C_1$-$C_4$) alkoxycarbonyl, di($C_1$-$C_4$ alkyl)amino, $OZ_1$ in which $Z_1$ represents ($C_1$-$C_4$) carboxyalkyl or ($C_1$-$C_4$) hydroxyalkyl, or $SZ_2$ in which $Z_2$ represents ($C_1$-$C_4$) hydroxyalkyl, ($C_2$-$C_4$) dihydroxyalkyl, ($C_1$-$C_4$) carboxyalkyl or ($C_1$-$C_4$) alkyl.

X denotes oxygen or the $NR_{11}$ group;

Y denotes oxygen or the $NR_{12}$ group, and

$R_{11}$ and $R_{12}$, independently of one another, denote hydrogen, halogen or $C_1$-$C_4$ lower alkyl; methylsulphonyl or phenylsulphonyl.

6. Method according to any one of Claims 1 to 5, characterised in that the quinone derivatives are chosen from the compounds of formulae:

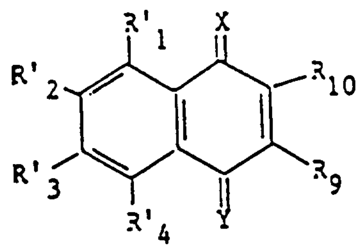

(V)

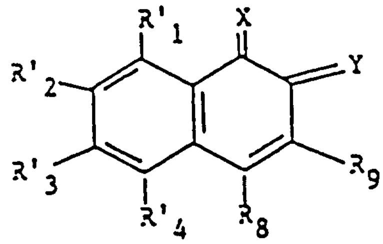

(VI)

in which formulae:

$R_8$, $R_9$ and $R_{10}$ have the meanings indicated in Claim 5; $R'_1$, $R'_2$, $R'_3$ and $R'_4$ have the meanings indicated for $R_6$, $R_7$, $R_8$, $R_9$ and $R_{10}$ in Claim 5,

X and Y having the same meanings as those indicated in Claim 5.

7. Method according to any one of Claims 1 to 5, characterised in that the quinone derivatives are chosen from the following compounds: 1,4-benzoquinone, 2-methoxy-1,4-benzoquinone, 2-methyl-1,4-benzoquinone, 2,6-dimethyl-1,4-benzoquinone, 2,3,5-trichloro-6-methyl-1,4-benzoquinone, 2-acetylamino-1,4-benzoquinone, 2-acetylamino-3,5-dimethyl-1,4-benzoquinone, 2-chloro-1,4-benzoquinone, tetrachloro-1,2-benzoquinone, 2,3-dimethoxy 1,4-benzoquinone, 2-β-carboxyethoxy-1,4-benzoquinone, 2-methoxymethyl-1,4-benzoquinone, 2-hydroxymethyl-1,4-benzoquinone, 2-β-hydroxyethylthio-1,4-benzoquinone, 2,5-bis-β-hydroxyethylthio-1,4-benzoquinone, 2-β,γ-dihydroxypropylthio-1,4-benzoquinone, 2-β-carboxyethylthio-1,4-benzoquinone, 2-carboxymethyl-1,4-benzoquinone, 2-β-hydroxyethylthio-6-methyl-1,4-benzoquinone, 2-methoxycarbonyl-3-methoxy-1,4-benzoquinone, 2-methoxycarbonyl-1,4-benzoquinone, 2-methylthio-1,4-benzoquinone, 2-dimethylamino-1,4-benzoquinone, 2-acetylamino-5-methoxy-1,4-benzoquinone, 2-(β-hydroxyethylthio)methyl-1,4-benzoquinone, 2-(methylthio)methyl-1,4-benzoquinone, 4,5-dimethoxy-1,2-benzoquinone, 4-methyl-5-chloro-1,2-benzoquinone, 4,5-dimethyl-1,2-benzoquinone, 2,3-dimethyl-1,4-benzoquinone, 2-β-hydroxyethoxy-1,4-benzoquinone, N-methylsulphonyl-1,4-benzoquinone monoimine, N-phenylsulphonyl-1,4-benzoquinone monoimine, 1,4-naphthoquinone, 1,2-naphthoquinone, 1,2-naphthoquinone-4-sulphonic acid, 2,3-dichloro-1,4-naphthoquinone, and N-2,6-trichloro-1,4-benzoquinone imine.

8. Method according to any one of Claims 1 to 7, characterised in that the aminoindole is present in the composition (A) in a concentration varying between 0.01 and 0.3 mol/litre.

9. Method according to any one of Claims 1 to 7, characterised in that the quinone derivative is present in the composition (B) in concentrations of between 0.005 and 1 mol/litre.

10. Method according to any one of Claims 1 to 9, characterised in that the pH of the compositions (A) and (B), independently of one another, is between 2 and 10.

11. Method according to Claim 10, characterised in that the pH of the composition (B) is acid.

12. Method according to any one of Claims 1 to 11, characterised in that the compositions (A) and (B) comprise an aqueous medium consisting of water or a mixture of water and a solvent.

13. Method according to any one of Claims 1 to 9, characterised in that the composition (A) and/or (B) consists of an anhydrous solvent medium.

14. Method according to any one of Claims 1 to 13, characterised in that the compositions (A) and (B) independently of one another contain anionic, cationic, nonionic or amphoteric surfactants or their mixtures, thickeners, perfumes, sequestrant agents, film-forming agents, treatment agents, dispersing agents, conditioners, preservatives, opacifying agents and agents for swelling keratinous fibres.

15. Method according to any one of Claims 1 to 14, characterised in that the compositions (A) and/or (B) contain other dyes chosen from direct dyes, oxidation dyes or couplers or so-called "rapid" oxidation dyes.

16. Method according to any one of Claims 1 to 15, characterised in that the composition (A) and/or (B) also contains quinone dyes of the class of benzoquinones, benzoquinone imines or benzoquinone diimines, naphthoquinones, naphthoquinone imines, naphthoquinone diimines which have potentials such that $\Delta E$ is higher than 470 mV.

17. Multicompartment device or dyeing kit, characterised in that it comprises the composition (A) as defined in any one of Claims 1 to 16, in a first compartment, and a composition (B) as defined in any one of Claims 1 to 16, in a second compartment.

18. Multicompartment device or dyeing kit, characterised in that it comprises a composition (A) as defined in any one of Claims 1 to 16, in a first compartment, the composition (B) as defined in any one of Claims 1 to 16, in a second compartment, at least one of the two compositions (A) and (B) comprising an anhydrous solvent medium, and a third compartment containing an aqueous medium for the dyeing and intended to be mixed just before use with the contents of one or other of the two compartments containing the anhydrous compositions (A) and/or (B).

**Patentansprüche**

1. Verfahren zur Färbung keratinischer Fasern,
dadurch **gekennzeichnet**, daß
man auf diese Fasern mindestens eine Zusammensetzung (A) aufträgt, die in einem zur Färbung geeigneten Milieu mindestens ein Aminoindol enthält, das verschieden ist von 2,3-Dimethyl-5-hydroxy-6-aminoindol und 2,3-Dimethyl-6-hydroxy-5-aminoindol, wobei vor oder nach der Aufbringung der Zusammensetzung (A) die Aufbringung einer Zusammensetzung (B) erfolgt, die, in einem zur Färbung geeigneten Milieu, mindestens ein Chinonderivat enthält, ausgewählt aus o- oder p-Benzochinonen, o- oder p-Benzochinonmonoiminen oder -diiminen, o- oder p-Benzochinonsulfonimiden, $\alpha,\omega$-Alkylenbis-1,4-benzochinonen, 1,2- oder 1,4-Naphthochinonen, 1,2- oder 1,4-Naphthochinonmonoiminen oder -diiminen, wobei die Aminoindole und die Chinonderivate so ausgewählt sind, daß die Redoxpotential-differenz $\Delta E$ zwischen dem Redoxpotential $E_i$ der Aminoindole, bestimmt durch Voltametrie bei pH 7 in Phosphat-Milieu an einer Elektrode aus glasartigem Kohlenstoff,
und dem Redoxpotential $E_q$ der Chinonderivate, bestimmt durch Polarographie bei pH 7 in Phosphat-Milieu an einer Quecksilberelektrode gegenüber einer gesättigten Kalomel-Elektrode, beträgt:

$$\Delta E = E_i - E_q \leqq 470 \text{ mV}.$$

2. Verfahren gemäß Anspruch 1,
dadurch **gekennzeichnet**, daß
die Aminoindole aus Verbindungen der Formel:

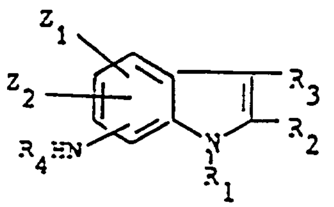

(I)

worin gilt:
$R_1$ bedeutet ein Wasserstoffatom oder eine lineare oder verzweigte $C_{1-4}$-Alkylgruppe;
$R_2$ und $R_3$ bedeuten ein Wasserstoffatom, eine $C_{1-4}$-Alkylgruppe, eine Carboxyl- oder $C_{1-4}$-Alkoxycarbonylgruppe;
$R_4$ bedeutet ein Wasserstoffatom oder eine $C_{1-4}$-Alkyl- oder $C_{1-4}$-Hydroxyalkyl- oder $C_{2-4}$-Polyhydroxyalkyl-, Acetyl-, $C_{1-6}$-Aminoalkylgruppe, deren Aminrest gegebenenfalls durch einen $C_{1-4}$-Alkylrest mono- oder disubstituiert sein kann ausgenommen 2,3-Dimethyl-5-hydroxy-6-aminoindol und 2,3-Dimethyl-6-hydroxy-5-aminoindol,
$Z_1$ und $Z_2$, gleich oder verschieden, stellen ein Wasserstoffatom, eine $C_{1-4}$-Alykl-, Hydroxy-, Halogen- oder $C_{1-4}$-Alkoxygruppe dar,
wobei die $R_4$NH-Gruppe die Positionen 4, 5, 6 oder 7 des Benzolkerns einnimmt, sowie aus deren Salzen ausgewählt sind.

3. Verfahren gemäß Anspruch 1 oder 2,
dadurch **gekennzeichnet**, daß
die Aminoindole ausgewählt sind aus:
5-Aminoindol, 6-Aminoindol, 7-Aminoindol, 6-N-β-Hydroxyethylaminoindol, 6-N-β-hydroxyethylamino-1-methylindol, 6-Methylaminoindol, 6-Amino-N-methylindol, 6-Amino-2-carboxyindol, 4-Amino-2,3-dimethylindol, 6-Amino-2,3-dimethylindol, 7-Amino-2,3-dimethylindol, 6-Amino-3-ethyl-2-methylindol, 7-Amino-3-ethyl-2-methylindol, 6-Amino-3-methylindol, 6-Amino-2-methylindol, 6-Amino-2-ethoxycarbonylindol, 4-Aminoindol, 5-Amino-6-methoxy-2,3-dimethylindol, 6-Amino-5-methoxy-2,3-dimethylindol, 5-Amino-N-methylindol, 6-N-(β,γ-Dihydroxypropyl)aminoindol, 6-Amino-2,3,4,5-tetramethylindol, 6-Amino-5-chlor-2,3-dimethylindol, 6-Amino-5-ethyl-2,3-dimethylindol, 6-Amino-2,3,4-trimethylindol, 6-Amino-5-hydroxy-2-methylindol, 4-Methylaminoindol, 4-Amino-N-methylindol, 6-Amino-2,3,7-trimethylindol, 6-Amino-2,3,5-trimethylindol, 5-Acetylamino-6-methoxy-2,3-dimethylindol, 6-Amino-4-methylindol, 6-Amino-5-methylindol, 4-Amino-7-methylindol, 6-Amino-7-ethyl-3-methylindol, 6-Amino-5,7-dimethylindol, 6-Amino-5,7-diethylindol, 7-Amino-5-methyl-2-ethoxycarbonylindol, 7-Amino-5-chlor-2-ethoxycarbonylindol, 7-Amino-5-ethoxy-2-ethoxycarbonylindol, 7-Amino-5-methoxy-2-ethoxycarbonylindol, 7-(4'-Dimethylamino-1'-methylbutyl)amino-5-methoxyindol, 7-(4'-Dimethylaminobutyl)-amino-5-methoxyindol, 6-Amino-5-fluorindol, 6-Amino-5-fluor-1-sec.butylindol, 6-Amino-5-fluor-1-n-propylindol, 6-Amino-2-methoxycarbonyl-5-methoxy-N-methylindol, 6-Amino-5-methoxy-2-methoxycarbonylindol, 6-Amino-5-methoxy-2-ethoxycarbonylindol, 6-Amino-5-methoxy-2-carboxyindol, 6-Amino-5-hydroxy-1,2-dimethylindol und 6-Amino-4-methoxy-2-methoxycarbonylindol.

4. Verfahren gemäß jedem der Ansprüche 1 bis 3,
dadurch **gekennzeichnet**, daß
die Chinonderivate aus Verbindungen der Formeln (II) und (II') ausgewählt sind:

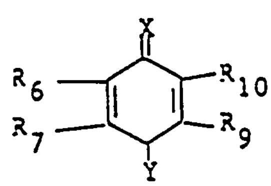

(II)

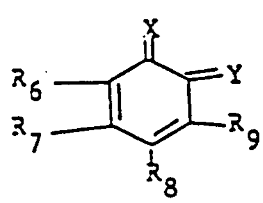

(II')

worin gilt:
X bezeichnet Sauerstoff oder eine $NR_{11}$-Gruppe;
Y bezeichnet Sauerstoff oder eine $NR_{12}$-Gruppe,

wobei $R_{11}$ und $R_{12}$, gleich oder verschieden, Wasserstoff, Halogen, einen $C_{1-4}$-Alkyl-, $C_{1-4}$-Hydroxyalkyl-, $C_{1-4}$-Alkylsulfonyl- oder einen Phenylsulfonylrest bedeuten;
$R_6$, $R_7$, $R_8$, $R_9$ und $R_{10}$ bezeichnen, unabhängig voneinander, Wasserstoff, einen $C_{1-4}$-Alkylrest, eine Carboxyl-, $C_{1-4}$-Alkylcarbonyl-, $C_{1-4}$-Alkoxycarbonyl-, $C_{1-4}$-Alkoxymethyl-, $C_{1-4}$-Alkylthiomethyl-, $C_{1-4}$-Hydroxyalkylthiomethyl-, $C_{1-4}$-Hydroxyalkylsulfinylgruppe, eine Gruppe

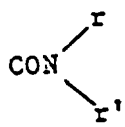

(worin r und r', unabhängig voneinander,
Wasserstoff oder einen $C_{1-4}$-Alkylrest darstellen), einen $C_{1-4}$-Carboxyalkylrest, Halogen, einen $C_{1-4}$-Hydroxyalkylrest, einen mit einer oder zwei $C_{1-4}$-Alkyl- oder -Hydroxyalkylgruppen substituierten oder nicht substituierten Aminorest, einen $C_{2-6}$-Acylaminorest, $SO_3M$, worin M Wasserstoff, K oder Na darstellt, eine gegebenenfalls substituierte Sulfoxid-, Sulfon- oder Sulfonamidgruppe, oder auch einen $OZ_1$-Rest, worin $Z_1$ Wasserstoff, eine $C_{1-4}$-Alkyl-, $C_{1-4}$-Hydroxyalkyl-, $C_{1-4}$-Carboxyalkyl-, Phenylgruppe sein kann, die gegebenenfalls mit einem $C_{1-4}$-Alkoxyrest substituiert ist, oder auch einen Rest $-SZ_2$, worin $Z_2$ eine $C_{1-4}$-Alkyl-, $C_{1-4}$-Hydroxyalkyl-, $C_{2-4}$-Dihydroxyalkyl-, $C_{1-4}$-Carboxyalkylgruppe ist,
wobei $R_6$ und $R_7$ mit den Kohlenstoffatomen, an die sie gebunden sind, die folgende zyklische Gruppe bilden können:

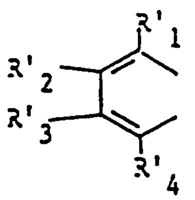

(III)

worin gilt:
$R'_1$, $R'_2$, $R'_3$ und $R'_4$ haben die oben für $R_6$, $R_7$, $R_8$, $R_9$, $R_{10}$ angegebenen Bedeutungen, solange sie keine Zyklen bilden.

**5.** Verfahren gemäß jedem der Ansprüche 1 bis 4,
dadurch **gekennzeichnet**, daß
die Chinonderivate aus den Benzochinonen der Formeln ausgewählt sind:

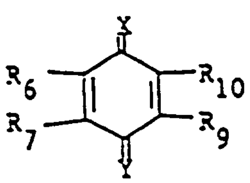

(II)

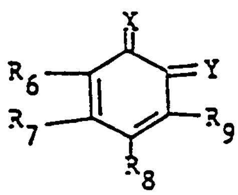

(II')

in denen gilt:
$R_6$, $R_7$, $R_8$, $R_9$, $R_{10}$ bezeichnen, unabhängig voneinander, Wasserstoff, einen $C_{1-4}$-Niedrigalkyl-, $C_{1-4}$-Niedrigalkoxy-, Halogen-, $C_{2-6}$-Acylamino-, $-SO_3M$-, $C_{1-4}$-Alkoxymethyl-, $C_{1-4}$-Carboxyalkyl-, $C_{1-4}$-Alkoxycarbonyl-, $C_{1-4}$-Dialkylamino-, $OZ_1$-Rest, worin $Z_1$ einen $C_{1-4}$-Carboxyalkyl- oder $C_{1-4}$-Hydroxyalkylrest darstellt, sowie einen $SZ_2$-Rest, worin $Z_2$ einen $C_{1-4}$-Alkyl-, $C_{1-4}$-Hydroxyalkyl-, $C_{2-4}$-Dihydroxyalkyl- oder $C_{1-4}$-Carboxyalkylrest darstellt;
X bedeutet Sauerstoff oder die $NR_{11}$-Gruppe;
Y bedeutet Sauerstoff oder die $NR_{12}$-Gruppe,
wobei $R_{11}$ und $R_{12}$, unabhängig voneinander, Wasserstoff, Halogen oder einen $C_{1-4}$-Niedrigalkyl-,

Methylsulfonyl- oder Phenylsulfonylrest darstellen.

**6.** Verahren gemäß jedem der Ansprüche 1 bis 5,
dadurch **gekennzeichnet**, daß
die Chinonderivate aus den Verbindungen der Formeln ausgewählt sind:

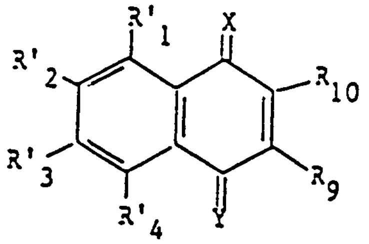
(V)

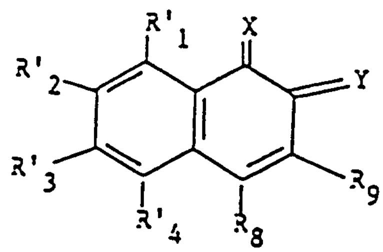
(VI)

in denen gilt:
$R_8$, $R_9$ und $R_{10}$ haben die in Anspruch 5 angegebenen Bedeutungen; $R'_1$, $R'_2$, $R'_3$ und $R'_4$ haben die für $R_6$, $R_7$, $R_8$, $R_9$ und $R_{10}$ die in Anspruch 5 angegebenen Bedeutungen;
X und Y haben dieselben Bedeutungen wie die in Anspruch 5 angegebenen.

**7.** Verfahren gemäß jedem der Ansprüche 1 bis 5,
dadurch **gekennzeichnet**, daß
die Chinonderivate aus den folgenden Verbindungen ausgewählt sind:
1,4-Benzochinon, 2-Methoxy-1,4-benzochinon, 2-Methyl-1,4-benzochinon, 2,6-Dimethyl-1,4-benzochinon, 2,3,5-Trichlor-6-methyl-1,4-benzochinon, 2-Acetylamino-1,4-benzochinon, 2-Acetylamino-1,4-benzochinon, 2-Acetylamino-3,5-dimethyl-1,4-benzochinon, 2-Chlor-1,4-benzochinon, Tetrachlor-1,2-benzochinon, 23-Dimethoxy-1,4-benzochinon, 2-β-Carboxyethoxy-1,4-benzochinon, 2-Methoxymethyl-1,4-benzochinon, 2-Hydroxymethyl-1,4-benzochinon, 2-β-Hydroxyethylthio-1,4-benzochinon, 2,5-Bis-β-hydroxyethylthio-1,4-benzochinon, 2-β-γ-Dihydroxypropylthio-1,4-benzochinon, 2-β-Carboxyethylthio-1,4-benzochinon, 2-Carboxymethyl-1,4-benzochinon, 2-β-Hydroxyethylthio-6-methyl-1,4-benzochinon, 2-Methoxycarbonyl-3-methoxy-1,4-benzochinon, 2-Methoxycarbonyl-1,4-benzochinon, 2-Methylthio-1,4-benzochinon, 2-Dimethylamino-1,4-benzochinon, 2-Acetylamino-5-methoxy-1,4-benzochinon, 2-(β-Hydroxyethylthio)methyl-1,4-benzochinon, 2-(Methylthio)methyl-1,4-benzochinon, 4,5-Dimethoxy-1,2-benzochinon, 4-Methyl-5-chlor-1,2-benzochinon, 4,5-Dimethyl-1,2-benzochinon, 2,3-Dimethyl-1,4-benzochinon, 2-β-Hydroxyethoxy-1,4-benzochinon, N-Methylsulfonyl-1,4-benzochinonmonoimin, N-Phenylsulfonyl-1,4-benzochinonmonoimin, 1,4-Naphthochinon, 1,2-Naphthochinon, 1,2-Naphthochinon-4-sulfonsäure, 2,3-Dichlor-1,4-naphthochinon, N-2,6-Trichlor-1,4-benzochinonimin.

**8.** Verfahren gemäß jedem der Ansprüche 1 bis 7,
dadurch **gekennzeichnet**, daß
das Aminoindol in der Zusammensetzung (A) in einer Konzentration von 0,01 bis 0,3 Mol/l vorhanden ist.

**9.** Verfahren gemäß jedem der Ansprüche 1 bis 7,
dadurch **gekennzeichnet**, daß
das Chinonderivat in der Zusammensetzung (B) in Konzentrationen von 0,005 bis 1 Mol/l vorhanden ist.

**10.** Verfahren gemäß jedem der Ansprüche 1 bis 9,
dadurch **gekennzeichnet**, daß
der pH der Zusammensetzungen (A) und (B) unabhängig voneinander 2 bis 10 beträgt.

**11.** Verfahren gemäß Anspruch 10,
dadurch **gekennzeichnet**, daß
der pH der Zusammensetzung (B) im sauren Bereich liegt.

**12.** Verfahren gemäß jedem der Ansprüche 1 bis 11, dadurch **gekennzeichnet**, daß die Zusammensetzungen (A) und (B) ein wässriges Milieu aus Wasser oder einer Mischung aus Wasser und einem Lösungsmittel enthalten.

**13.** Verfahren gemäß jedem der Ansprüche 1 bis 9, dadurch **gekennzeichnet**, daß die Zusammensetzung (A) und/oder (B) aus einem wasserfreien Lösungsmittelmilieu zusammengesetzt sind.

**14.** Verfahren gemäß jedem der Ansprüche 1 bis 13, dadurch **gekennzeichnet**, daß die Zusammensetzungen (A) und (B) unabhängig voneinander anionische, kationische, nicht-ionische, amphotere oberflächenaktive Mittel oder deren Mischungen, Verdickungsmittel, Parfüm-Produkte, Sequestriermittel, filmbildende Mittel, Behandlungsmittel, Dispergiermittel, Konditioniermittel, Konservierungsstoffe, opak machende Mittel, Auflockerungsmittel für keratinische Fasern enthalten.

**15.** Verfahren gemäß jedem der Ansprüche 1 bis 14, dadurch **gekennzeichnet**, daß die Zusammensetzungen (A) und/oder (B) weitere Farbstoffe enthalten, ausgewählt aus Direktfarbstoffen, Oxidationsfarbstoffen, "Rapidfarbstoffe" genannten Kupplern oder Oxidationsfarbstoffen.

**16.** Verahren gemäß jedem der Ansprüche 1 bis 15, dadurch **gekennzeichnet**, daß die Zusammensetzung (A) und/oder (B) auch Chinon-Farbstoffe der Familie der Benzochinone, Benzochinonimine oder -diimine, Naphthochinone, Naphthochinonimine, Naphthochinindiimine enthalten, deren Potentiale so bemessen sind, daß $\Delta E$ oberhalb 470 mV liegt.

**17.** Vorrichtung aus mehreren Teilstücken oder Kit zur Färbung, dadurch **gekennzeichnet**, daß sie in einem ersten Teilstück die in jedem der Ansprüche 1 bis 16 definierte Zusammensetzung (A) und in einem zweiten Teilstück eine in jedem der Ansprüche 1 bis 16 definierte Zusammensetzung (B) umfassen.

**18.** Vorrichtung aus mehreren Teilstücken oder Kit zur Färbung, dadurch **gekennzeichnet**, daß sie in einem ersten Teilstück eine in jedem der Ansprüche 1 bis 16 definierte Zusammensetzung (A), in einem zweiten Teilstück die in jedem der Ansprüche 1 bis 16 definierte Zusammensetzung (B), wobei mindestens eine der beiden Zusammensetzungen (A) und (B) ein wasserfreies Lösungsmittelmilieu enthält, sowie ein drittes Teilstück umfassen, das ein wässriges Milieu zur Färbung enthält und dazu bestimmt ist, kurz vor dem Gebrauch mit dem Inhalt des einen oder anderen der beiden Teilstücke vermischt zu werden, die die wasserfreien Zusammensetzungen (A) und/oder (B) enthalten.